# EUROPEAN PATENT APPLICATION

(11) **EP 2 876 157 A1**
(43) Date of publication of application: **27.05.2015**
(21) Application number: 13194450.6
(22) Date of filing: 26.11.2013
(51) Int. Cl.: C12N 9/18, C08J 11/10, C08L 67/02

(54) **Degradation of polyesters under anaerobic conditions**

(71) Applicant: BASF SE, 67056 Ludwigshafen (DE)
(72) Inventor: The designation of the inventor has not yet been filed
(74) Representative: Dick, Alexander

(57) **Abstract**

The present invention relates to the field of degradation of polyesters in connection with, e.g., waste management in biogas producing plants. In particular, the present invention concerns a method for the degradation of a polyester comprising the steps of contacting the polyester with at least one esterase and/or lipase and/or cutinase, preferably comprised in a host cell, wherein the said at least one esterase and/or lipase and/or cutinase is obtainable from a microorganism and incubating the said polyester with the said at least one esterase and/or lipase and/or cutinase for a time and under conditions sufficient to allow for degradation of the polyester. Moreover, the invention encompasses the use of at least one esterase and/or lipase for degrading a polyester, wherein the said at least one esterase and/or lipase is obtainable from a microorganism. Further provided are a polynucleotide encoding an esterase, a vector comprising said polynucleotide, a host cell comprising said polynucleotide or said vector as well as an esterase polypeptide. Moreover, the present invention provides a composition comprising the said esterase polypeptide and a further agent which improves the degradation of degrading a polyester.

## Description

The present invention relates to the field of degradation of polyesters in connection with, e.g., waste management in biogas producing plants. In particular, the present invention concerns a method for the degradation of a polyester comprising the steps of contacting the polyester with at least one esterase and/or lipase and/or cutinase, preferably comprised in a host cell, wherein the said at least one esterase and/or lipase and/or cutinase is obtainable from a microorganism and incubating the said polyester with the said at least one esterase and/or lipase and/or cutinase for a time and under conditions sufficient to allow for degradation of the polyester. Moreover, the invention encompasses the use of at least one esterase and/or lipase for degrading a polyester, wherein the said at least one esterase and/or lipase is obtainable from a microorganism. Further provided is a polynucleotide encoding an esterase, a vector comprising said polynucleotide, a host cell comprising said polynucleotide or said vector as well as an esterase polypeptide. Moreover, the present invention provides a composition comprising the said esterase polypeptide and a further agent which improves the degradation of degrading a polyester.

Biodegradable polyesters, such as aliphatic-aromatic polyesters, are used as biodegradable plastics for various purposes including packaging materials and foils such as agriculture foils. Accordingly, theses polyesters need to be handled and degraded as a part of waste management processes in, e.g., waste recycling plants or biogas plants. Degradation processes in, e.g., biogas plants, however, require an efficient degradation of the waste components even under anaerobic conditions.

The degradation of aliphatic-aromatic polyesters under aerobic conditions is well studied and proven. However, degradation of such polyesters under anaerobic conditions (e.g. in biogas plants) is still limited. Nevertheless, the degradation of aliphatic-aromatic polyesters used in, e.g., packaging material such as biowaste bags, is of great interest since this polyester is produced in industrial scale and biodegradation under anaerobic condition needs to be improved.

Considerably little knowledge exists about enzymes that may facilitate the degradation of aliphatic-aromatic polyesters although especially this knowledge might help to improve and boost the degradation of such polyesters not only in aerobic but also in anaerobic conditions.

Literature studies showed that species from the genus Clostridium might play a role in terms of anaerobic degradation of polyesters (Abou-Zeid 2001, J. Biotechnol 86: 113-126; Mueller 2006, Process Biochemistry 41: 2124-2128). Apparently, enzymatic degradation of aliphatic-aromatic polyesters under anaerobic conditions, such as those in biogas plants, has not yet been reported.

The technical problem underlying the present invention can be seen as the provision of means and methods for complying with the aforementioned needs. The said technical problem is solved by the embodiments characterized in the claims and herein below.

Thus, the present invention relates to a method for the degradation of a polyester comprising the steps of:
a) contacting the polyester with at least one esterase and/or lipase and/or cutinase, wherein the said at least one esterase and/or lipase and/or cutinase is obtainable from a microorganism; and
b) incubating the said polyester with the said at least one esterase and/or lipase and/or cutinase for a time and under conditions sufficient to allow for degradation of the polyester.

The term "degradation" as used herein refers to hydrolysis or partial hydrolysis of a polyester, preferably, as specified elsewhere herein. Preferably, in accordance with the present invention degradation means that at least a statistically significant portion of the polyester is hydrolyzed or partially hydrolyzed. Statistic significance can be determined by well known statistical test by the skilled person without further ado. More preferably, it is envisaged that all of the aliphaticaromatic polyester subjected to the method of the invention is entirely or partially hydrolyzed.

The term "polyester" as used herein encompasses aliphatic polyesters as well as aliphaticaromatic polyesters. Preferably, the polyester is a biodegradable polyester. Such biodegradable polyesters may be artificially synthesized or may occur naturally. The polyester according to the present invention may be subjected to the method of the present invention in any form, e.g., as free polyester molecules, fibers, fabrics, as part of compositions or immobilized on or comprised in items, such as biodegradable packaging material.

Aliphatic polyesters as used herein are polymers which contain the ester functional group in the main chain, wherein the ester groups in the main chain are bridged by aliphatic residues. Aliphatic polyesters can be homopolymers derived from monomers containing a carboxyl group and a hydroxyl group which are bridged by an aliphatic residue, or copolymers which are derived from a diol compound wherein the hydroxy groups are bridged by an aliphatic residue and a dicarboxylic acid wherein the carbonxyl groups are bridged by an aliphatic residue. Examples of aliphatic homopolymeric polyesters are polyglycolide (PGA), polylactic acid (PLA), polycaprolactone (PCL), polyhydroxyalkanoates (PHA), e.g., poly-3-hydroxybutyrate (PHB). Examples of aliphatic copolymeric polyesters are polyethylene adipate (PEA), polybutylene succinate (PBS), poly(3-hydroxybutyrate-co-hydroxyvalerate (PHBV). Preferably, the aliphatic polyesters referred to herein are obtained by a condensation reaction and comprise one or more aliphatic dicarboxylic acids, one or more aliphatic diols and one or more polyfunctional compound. The aliphatic dicarboxylic acid is, preferably, a C4-36 dicarboxylic acid. The aliphatic diol is, preferably, a C2-8 alylene diol or a C2-C6 oxyalkylene. Preferably, the aliphatic polyester comprises 65 to 100 mol% succinic acid or their respective derivatives, 0 to 35 mol% C5-C36 dicarboxlic acid or their respectives acid derivatives or as a mixture thereof, 98 to 100 mol% C2-C8 alkylene diol or C2-C6 oxyalcylene diol and at least 0 to 2 mol% isocyanate-, isocyanurate-, oxazoline-, or epoxidegroups or at least three alcoholic- or carboxylic acid-groups. Preferably, the aliphatic polyester is selected from the group consisting of: poly(butylene succinate alipate((PBSA), polybutylene succinate (PBS), polycarbrolactone (PCL), and Polybutylenesuccinatesebacate (PBSSe). The naturally occurring aliphatic polyester as used herein refers to polyesters made from naturally produced macromolecules or renewable resources. Preferably, the natural polyesters are obtained by a condensation reaction in the presence of a catalyst and may be polylactic acid (PLA), polyhydroxyalkanoate (PHA), polyglycolide (PGA), starches, cellulose, chitin or gelatin for example.

The term "aliphatic-aromatic polyester" as used herein refers to co-polymers of aliphatic polyesters and aromatic polyesters. Preferably, the aliphatic-aromatic polyesters are obtained by a condensation reaction and comprise one ore more aliphatic dicarboxylic acids, one ore more aromatic dicarboxylic acids, one or more aliphatic diols and one or more polyfunctional compound.

Particularly preferred are aliphatic-aromatic polyesters based on aliphatic and aromatic dicarboxylic acids and on aliphatic dihydroxy compound, and any of the aliphatic polyesters made of aliphatic dicarboxylic acids and of aliphatic diols. A feature common to said polyesters is that they are biodegradable to DIN EN 13432 (under aerobic conditions). Mixtures of a plurality of these polyesters are of course also suitable.

Among these particularly preferred polyesters are polyesters which comprise, as essential components,
A) an acid component made of:
   a1) from 30 to 100 mol% of at least one aliphatic dicarboxylic acid or ester-forming derivatives thereof, or a mixture thereof,
   a2) from 0 to 70 mol% of at least one aromatic dicarboxylic acid or ester-forming derivative thereof, or a mixture thereof,
      and
B) from 98 to 102 mol%, based on acid component A, of a diol component B selected from at least one C2-C12-alkanediol or a mixture thereof
   and
C) from 0.01 to 3% by weight, based on components A and B, of a component C selected from
   c1) a compound having at least three groups capable of ester formation or of amide formation,
   c2) a di- or polyisocyanate,
   c3) a di- or polyepoxide,
   or a mixture made of c1) to c3).

Compounds which can be used as aliphatic acids or as the corresponding derivatives a1 are generally those having from 2 to 18 carbon atoms, preferably from 4 to 10 carbon atoms. They can be either linear or branched compounds. In principle, however, it is also possible to use dicarboxylic acids having a larger number of carbon atoms, for example having up to 30 carbon atoms.

Examples that may be mentioned are: oxalic acid, malonic acid, succinic acid, glutaric acid, 2-methylglutaric acid, 3-methylglutaric acid, α-ketoglutaric acid, adipic acid, pimelic acid, azelaic acid, sebacic acid, brassylic acid, fumaric acid, 2,2-dimethylglutaric acid, suberic acid, diglycolic acid, oxaloacetic acid, glutamic acid, aspartic acid, itaconic acid, and maleic acid. It is possible here to use the dicarboxylic acids or ester-forming derivatives thereof, individually or in the form of a mixture made of two or more thereof.

It is preferable to use succinic acid, adipic acid, azelaic acid, sebacic acid, brassylic acid, or respective ester-forming derivatives thereof, or a mixture thereof. It is particularly preferable to use succinic acid, adipic acid, or sebacic acid, or respective ester-forming derivatives thereof, or a mixture thereof. Succinic acid, azelaic acid, sebacic acid, and brassylic acid have the additional advantage that they are obtainable from renewable raw materials]

The aromatic dicarboxylic acids or ester-forming derivatives thereof a2 can be used individually or in the form of a mixture made of two or more thereof. It is particularly preferable to use terephthalic acid or ester-forming derivatives thereof, e.g. dimethyl terephthalate.

The diols B are generally selected from branched or linear alkanediols having from 2 to 12 carbon atoms, preferably from 4 to 6 carbon atoms, or from cycloalkanediols having from 5 to 10 carbon atoms.

Examples of suitable alkanediols are ethylene glycol, 1,2-propanediol, 1,3-propanediol, 1,2-butanediol, 1,4-butanediol, 1,5-pentanediol, 2,4-dimethyl-2-ethylhexane-1,3-diol, 2,2-dimethyl-1,3-propanediol, 2-ethyl-2-butyl-1,3-propanediol, 2-ethyl-2-isobutyl-1,3-propanediol, 2,2,4-trimethyl-1,6-hexanediol, and in particular ethylene glycol, 1,3-propanediol, 1,4-butanediol, and 2,2-dimethyl-1,3-propanediol (neopentyl glycol); cyclopentanediol, 1,4-cyclohexanediol, 1,2-cyclohexanedimethanol, 1,3-cyclohexanedimethanol, 1,4-cyclohexanedimethanol, and 2,2,4,4-tetramethyl-1,3-cyclobutanediol. Particular preference is given to 1,4-butanediol, particularly in combination with adipic acid as component a1), and 1,3-propanediol, particularly in combination with sebacic acid as component a1). 1,3-Propanediol also has the advantage that it is obtainable in the form of renewable raw material. It is also possible to use a mixture of various alkanediols.

Particular preference is given to the following aliphatic-aromatic polyesters: polybutylene adipate terephthalate (PBAT), polybutylene sebacate terephthalate (PBSeT), and polybutylene succinate terephthalate (PBST) and commercially available products such as ecoflex® (BASF SE) and Eastar® Bio, and Origo-Bi® (Novamont).

Contacting as used herein refers to bringing into physical proximity the said at least one esterase, and/or lipase and/or cutinase and the polyester such that the enzyme can bind to the polyester and convert it by hydrolysis. Said contacting can be, preferably, carried out by contacting at least one purified or partially purified esterase, lipase or cutinase polypeptides or a composition comprising at least one esterase and at least one lipase, at least one esterase and at least one cutinase, at least one cutinase and at least one lipase or at least one esterase, at least one lipase and at least one cutinase with the polyesters. Also preferably, contacting may be carried out by contacting a host cell expressing said at least one esterase, lipase or cutinase polypeptide or expressing a combination of at least one esterase and at least one lipase, at least one esterase and at least one cutinase, at least one cutinase and at least one lipase or at least one esterase, at least one lipase and at least one cutinase with the polyester. Moreover, host cell combinations may be applied wherein said combinations comprise at least two host cells selected from the group consisting of: A host cell expressing at least one esterase, a host cell expressing at least one lipase and a host cell expressing at least one cutinase.

Thus, in a preferred embodiment of the method of the invention, said esterase and/or lipase and/or cutinase is comprised in a host cell. Such a host cell to be applied in the method of the present invention can be a host cell which has been genetically engineered to produce the esterase and/or lipase and/or cutinase polypeptides and, preferably, a host cell as specified elsewhere herein. Alternatively, host cells may be applied which naturally produce the said esterase and/or lipase and/or cutinase polypeptides. Preferably, the aforementioned host cells can be used as booster cultures in, e.g., biogas production reactors or waste degradation reactors.

The term "at least one" as used herein means that one or more, e.g., two, three, four, five or even more of the mentioned enzymes can be applied in the method of the invention. For example, it may be envisaged that two different esterases and/or two different lipases and/or two different cutinases are applied together in the method of the invention.

The term "esterase" as used herein refers to an enzyme which is capable of hydrolyzing ester compounds and, in particular, polyesters. Esterases are also sometimes referred to as hydrolases (E.C. 3.1.-.-). Lipid-hydrolyzing esterases are also called lipases (see below). The esterase to be used in accordance with the method of the present invention, preferably, exerts its hydrolyzing activity under anaerobic conditions. Typically, such esterases can be found in anaerobic microorganisms such as Clostridium species. The esterases of the present invention, preferably, have a temperature optimum between 20°C and 55°C and a pH optimum at a pH value which is between pH 3.5 to 8.5. Preferably, the esterase to be applied in accordance with the present invention is encoded by a polynucleotide comprising a nucleic acid sequence selected from the group consisting of:
a) a nucleic acid sequence as shown in SEQ ID NO: 1, 3 or 5;
b) a nucleic acid sequence encoding a polypeptide having an amino acid sequence as shown in SEQ ID NO: 2, 4 or 6;
c) a nucleic acid sequence encoding a polypeptide having esterase activity, said nucleic acid sequence having a nucleic acid sequence being at least 70% identical to the nucleic acid sequence shown in SEQ ID No: 1, 3 or 5;
d) a nucleic acid sequence encoding a polypeptide having esterase activity, said polypeptide having an amino acid sequence being at least 70% identical to the amino acid sequence shown in SEQ ID No: 2, 4 or 6; and
e) a nucleic acid sequence encoding a polypeptide having esterase activity, said nucleic acid sequence being capable of hybridizing to the nucleic acid sequence of any one of a) to d).

The term "polynucleotide" as used herein refers to single- or double-stranded DNA molecules as well as to RNA molecules. Encompassed by the said term is genomic DNA, cDNA, hnRNA, mRNA as well as all naturally occurring or artificially modified derivatives of such molecular species. The polynucleotide may be, preferably, a linear or circular molecule. Moreover, in addition to the nucleic acid sequences encoding the aforementioned polypeptide, a polynucleotide of the present invention may comprise additional sequences required for proper transcription and/or translation such as 5'- or 3'-UTR sequences and, preferably, at least 500, preferably 200, more preferably 100 nucleotides of the sequence upstream of the 5' terminus of the coding region and at least 100, preferably 50, more preferably 20 nucleotides of the sequence downstream of the 3' terminus of the coding gene region. The polynucleotide of the present invention, preferably, differs from the naturally occurring polynucleotides encoding esterases in that it is codon-optimized for use in a certain host cell or organism. For codon-optimization, the codons in the polynucleotide are changed such that the codons which are normally used by the host cell or organism replace the codons used by the organism which is the source of the polynucleotide. Codon-optimized polynucleotides can be chemically synthesized or obtained via mutagenesis techniques such as site-directed mutagenesis without further ado. Furthermore, the polynucleotides of the present invention may encode fusion proteins wherein one partner of the fusion protein is a polypeptide being encoded by a nucleic acid sequence recited above. Such fusion proteins may comprise as additional part other enzymes for the degradation of aliphatic-aromatic polyesters, polypeptides for monitoring expression (e.g., green, yellow, blue or red fluorescent proteins, alkaline phosphatase and the like) or so called "tags" which may serve as a detectable marker or as an aid for purification purposes. Tags for the different purposes are well known in the art and comprise FLAG-tags, 6-histidine-tags (also referred to as "His"-tag), MYC-tags and the like.

Encompassed as polynucleotides according to the invention are also polynucleotides comprising nucleic acid sequences which are variants of the aforementioned specific sequences. Preferably, such a variant comprises a nucleic acid sequence which is at least 70%, at least 80%, at least 90%, at least 95%, at least 98% or at least 99% identical with SEQ ID NO: 1, 3 or 5 and, preferably, encodes an esterase having essentially the same enzymatic properties as the esterase encoded by SEQ ID NO: 1, 3 or 5 or an esterase having SEQ ID NO: 2, 4 or 6. Moreover, the variant may preferably comprise an amino acid sequence which is at least 70%, at least 80%, at least 90%, at least 95%, at least 98% or at least 99% identical with SEQ ID NO: 2, 4 or 6 and, preferably, encodes an esterase having essentially the same enzymatic properties as the esterase encoded by SEQ ID NO: 1, 3 or 5 or an esterase having SEQ ID NO: 2, 4 or 6. Sequence identity as used herein is, preferably, to be determined by way of alignment over the entire length of an amino acid or nucleic acid sequence or over a contiguous stretch of amino acids or nucleotides, respectively, said stretch being at least 50% in length of the reference sequence (e.g., SEQ ID NO: 1, 2, 3, 4, 5 or 6) to which a given sequences shall be compared. A preferred algorithm for determining the percentage of sequence identity is the Needleman and Wunsch algorithm (Needleman 1970, J. Mol. Biol. (48):444-453) which has been incorporated into the needle program in the EMBOSS software package (EMBOSS: The European Molecular Biology Open Software Suite, Rice 2000, Trends in Genetics 16(6), 276-277), using either a BLOSUM 45 or PAM250 scoring matrix for distantly related proteins, or either a BLOSUM 62 or PAM160 scoring matrix for closer related proteins, and a gap opening penalty of 16, 14, 12, 10, 8, 6, or 4 and a gap extension penalty of 0.5, 1, 2, 3, 4, 5, or 6. Guides for local installation of the EMBOSS package as well as links to WEB-Services can be found at http://emboss.sourceforge.net. A preferred, non-limiting example of parameters to be used for aligning two amino acid sequences using the needle program are the default parameters, including the EBLOSU M62 scoring matrix, a gap opening penalty of 10 and a gap extension penalty of 0.5. In yet another preferred embodiment, the percentage of identity between two nucleotide sequences is determined using the needle program in the EMBOSS software package, using the EDNAFULL scoring matrix and a gap opening penalty of 16, 14, 12, 10, 8, 6, or 4 and a gap extension penalty of 0.5,1, 2, 3, 4, 5, or 6. A preferred, non-limiting example of parameters to be used in conjunction for aligning two nucleic acid sequences using the needle program are the default parameters, including the EDNAFULL scoring matrix, a gap opening penalty of 10 and a gap extension penalty of 0.5. The nucleic acid and protein sequences of the present invention can further be used as a "query sequence" to perform a search against public databases to, for example, identify other family members or related sequences. Such searches can be performed using the BLAST series of programs (version 2.2) of Altschul et al. (Altschul 1990, J. Mol. Biol. 215:403-10). Esterase activity of a variant can be, preferably, tested as described in the accompanying Examples, below, e.g., by measuring conversion of 4-nitrophenyl butyrate, 4-nitrophenyl acetate, 4-nitrophenyl octanoate or an aliphatic-aromatic polyester such as the particularly preferred aliphatic-aromatic polyesters referred to herein. Moreover, a fluorescence based assay may be used applying, e.g., fluorescein diacetate or fluorescein dilaurate.

Preferably, variants as referred to herein also include those variants which are encoded by a nucleic acid which hybridizes specifically and, preferably, under stringent conditions, with a nucleic acid as shown in SEQ ID NO: 1, 3 or 5 or encoding an amino acid sequence as shown in SEQ ID NO: 2, 4 or 5. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 x sodium chloride/sodium citrate (= SSC) at approximately 45°C, followed by one or more wash steps in 0.2 x SSC, 0.1 % SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 × SSC (pH 7.2). If organic solvent is present in the abovementioned buffer, for example 50% formamide, the temperature under standard conditions is approximately 42°C. The hybridization conditions for DNA: DNA hybrids are, preferably, 0.1 × SSC and 20°C to 45°C, preferably between 30°C and 45°C. The hybridization conditions for DNA:RNA hybrids are, preferably, 0.1 × SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the polypeptides of the present invention. Conserved domains of the polypeptide of the present invention may be identified by a sequence comparison of the nucleic acid sequences of the polynucleotides or the amino acid sequences of the polypeptides of the present invention. As a template, DNA from anaerobic microorganisms such as bacteria may be used. Preferably, the said variants encode esterases having essentially the same enzymatic properties as the esterase encoded by SEQ ID NO: 1 or 3 or an esterase having SEQ ID NO: 2 or 4. The said esterase activity of a variant can be, preferably, tested as described in the accompanying Examples, below, e.g., by measuring conversion of 4-nitrophenyl butyrate, 4nitrophenyl acetate, 4-nitrophenyl octanoate or an aliphatic-aromatic polyester such as the particularly preferred aliphatic-aromatic polyesters referred to herein. Moreover, a fluorescence based assay may be used applying, e.g., fluorescein diacetate or fluorescein dilaurate.

The term "lipase" as used herein refers to an enzyme which is capable of hydrolyzing lipid ester compounds and, in particular, polyesters (E.C. 3.1.1.3). The lipase to be used in accordance with the method of the present invention, preferably, exerts its hydrolyzing activity under anaerobic conditions. Typically, such lipases can be found in anaerobic microorganisms such as Clostridium species. The lipases of the present invention, preferably, have a temperature optimum between 20°C and 55°C and a pH optimum at a pH value which is between pH 3.5 to 8.5.

The term "cutinase" as used herein refers to an enzyme which is capable of hydrolyzing cutin from plant cuticles and related compounds (E.C. 3.1.1.74). The cutinase to be used in accordance with the method of the present invention, preferably, exerts its hydrolyzing activity under anaerobic conditions. Typically, such cutinase can be found in anaerobic microorganisms such as Clostridium species. The cutinases of the present invention, preferably, have a temperature optimum between 20°C and 55°C and a pH optimum at a pH value which is between pH 3.5 to 8.5.

The term "anaerobic microorganism" as referred to in accordance with the present invention relates to any microorganism, e.g., single cellular organism, host cell, bacterium, or yeast, which does not require oxygen for growth. Anaerobic microorganisms are well known in the art and it can be tested without further ado whether a microorganism is an anaerobic microorganism by cultivating it under anaerobic conditions. Preferably, the anaerobic microorganism in accordance with the present invention is a bacterium. More preferably, said anaerobic microorganism is a Clostridium species and, most preferably, Clostridium botulinum or Clostridium hathewayi. An esterase from Clostridium botulinum as referred to in accordance with the present invention, for instance, may have an amino acid sequence as shown in SEQ ID NO: 2 or may be encoded by an nucleic acid sequence as shown in SEQ ID NO: 1. Esterases from Clostridium hathewayi as referred to in accordance with the present invention, for instance, may have an amino acid sequence as shown in SEQ ID NO: 4 or 6 or may be encoded by an nucleic acid sequence as shown in SEQ ID NO: 3 or 5.

The term "incubating" as used herein refers to maintaining the at least one esterase, and/or lipase, and or cutinase in physical proximity to the polyester such that degradation thereof occurs. Dependent on whether purified or partially purified enzymes are used or host cell culture, it may be required to further add either buffer solutions in which the purified or partially purified enzymes are enzymatically active or growth media which allow for propagation of the host cell culture. Suitable incubation conditions are, preferably, a temperature between 20°C and 55°C and a pH at a pH value which is between pH 3.5 to 8.5. Moreover, said incubating is, preferably, carried out under anaerobic conditions. Incubation can be carried out for any time and, preferably, until the entire polyesters are degraded. Typically, incubation may be carried for several hours to several days or weeks, and preferably, from 1 hour to 4 weeks or more, more preferably, from 5 to 10 days and most preferably, for at least 5, 6 or 7 days. Preferably, said incubation is carried out on large scale in an incubator under controlled conditions, e.g., in a waste recycling plant or a biogas plant.

It will be understood that the method of the present invention can be applied for various purposes requiring the degradation of polyesters. According to the desired purpose, the method of the present invention may comprise steps in addition to those explicitly mentioned above.

For example, in a biogas plant, in a further step, the monomers of the polyesters could be converted, e.g., by acidogenic bacteria, into carbon dioxide, hydrogen, ammonia and/or organic acids and the latter organic acids in turn may be converted, e.g., by methanogenic bacteria, into methane and carbon dioxide. Thus, the method of the invention aiming at hydrolysis of the alipatic-aromatic polyesters may comprise additional steps such as the acidogenesis, acetogenesis and methanogenesis in order to generate biogases. In a waste recycling plant, the generated monomers of the polyesters may be purified for other purposes as well.

The method of the invention can, however, also be applied in polyester modification processes where a polyesters, such as the particularly preferred aliphatic-aromatic polyesters referred to above, needs to be entirely or partially hydrolyzed.

Advantageously, it has been found in the studies underlying the present invention that esterases, lipases and cutinases from anaerobic microorganisms such as the Clostridial species can be preferably applied for the degradation of polyesters, such as alipatic-aromatic polyesters and, in particular Ecoflex® manufactured by BASF SE, under anaerobic conditions. In particular, nucleic acid and amino acid sequences for an esterase from Clostridium botulinum (SEQ ID NOs: 1 and 2) and an esterase from Clostridium hathewayi (SEQ ID NOs: 2 and 4) were provided. The aforementioned anaerobic conditions are typically required in biogas or waste recycling plants. Accordingly, thanks to the present invention biogas production and waste management can be significantly improved. Moreover, the method helps to facilitate polyester modification processes.

The explanations and definitions given for the terms above apply mutatis mutandis for the following embodiments of the invention.

The present invention encompasses, in general, the use of at least one esterase and/or lipase for degrading a polyester, wherein the said at least one esterase and/or lipase is obtainable from an anaerobic microorganism. Preferably, said degrading a polyester is carried out under anaerobic conditions. As specified above already, the said esterase and/or lipase and/or cutinase is, preferably, comprised in a host cell or in a combination of host cells.

The present invention relates to a polynucleotide comprising a nucleic acid sequence as defined hereinbefore, i.e., a polynucleotide comprising a nucleic acid sequence selected from the group consisting of:
a) a nucleic acid sequence as shown in SEQ ID NO: 1, 3 or 5;
b) a nucleic acid sequence encoding a polypeptide having an amino acid sequence as shown in SEQ ID NO: 2, 4 or 6;
c) a nucleic acid sequence encoding a polypeptide having esterase activity, said nucleic acid sequence having a nucleic acid sequence being at least 70% identical to the nucleic acid sequence shown in SEQ ID No: 1, 3 or 5;
d) a nucleic acid sequence encoding a polypeptide having esterase activity, said polypeptide having an amino acid sequence being at least 70% identical to the amino acid sequence shown in SEQ ID No: 2, 4 or 6; and
e) a nucleic acid sequence encoding a polypeptide having esterase activity, said nucleic acid sequence being capable of hybridizing to the nucleic acid sequence of any one of a) to d).

Preferably, the polynucleotide of the invention is operatively linked to an expression control sequence.

The term "expression control sequence" as used herein refers to a nucleic acid sequence which is capable of governing, i.e. initiating and controlling, transcription of a nucleic acid sequence of interest, in the present case the nucleic sequences recited above. Such a sequence usually comprises or consists of a promoter or a combination of a promoter and enhancer sequences. Preferably, the expression control sequence is active and/or derived from a host cell in which the polynucleotide shall be expressed. Accordingly, dependent on the host cell, the expression control sequence can be homologous or heterologous with respect to the nucleic acid sequence of interest to be expressed. Expression of a polynucleotide comprises transcription of the nucleic acid molecule, preferably, into a translatable mRNA. Additional regulatory elements may include transcriptional as well as translational enhancers. The following promoters and expression control sequences may be, preferably, used in an expression vector according to the present invention. The cos, tac, trp, tet, trp-tet, lpp, lac, lpp-lac, laclq, T7, T5, T3, gal, trc, ara, SP6, λ-PR or λ-PL promoters are, preferably, used in Gram-negative bacteria. For Gram-positive bacteria, promoters amy and SPO2 may be used. From yeast or fungal promoters ADC1, AOX1 r, GAL1, MFα, AC, P-60, CYC1, GAPDH, TEF, rp28, ADH are, preferably, used. Particularly preferred are promoters which enable the expression in host cells according to the present invention and those which can be applied in the method of the invention for degrading polyesters.

Also preferably, the polynucleotide of the present invention may further comprise a terminator sequence operatively linked to the nucleic acid sequence to be expressed. The said terminator is a nucleic acid sequence which is capable of terminating transcription. These sequences will cause dissociation of the transcription machinery from the nucleic acid sequence to be transcribed. Preferably, the terminator shall be active in the host cell of the invention or those used in the method of the invention. Suitable terminators are known in the art.

The term "operatively linked" as used herein means that the expression control sequence and the nucleic acid sequence of interest are linked so that the expression of the said nucleic acid sequence of interest can be governed by the said expression control sequence, i.e. the expression control sequence shall be functionally linked to the said nucleic acid sequence to be expressed. Accordingly, the expression control sequence and, the nucleic acid sequence to be expressed may be physically linked to each other, e.g., by inserting the expression control sequence at the 5'end of the nucleic acid sequence to be expressed. Alternatively, the expression control sequence and the nucleic acid to be expressed may be merely in physical proximity so that the expression control sequence is capable of governing the expression of at least one nucleic acid sequence of interest. The expression control sequence and the nucleic acid to be expressed are, preferably, separated by not more than 500 bp, 300 bp, 100 bp, 80 bp, 60 bp, 40 bp, 20 bp, 10 bp or 5 bp.

The present invention further relates to a vector comprising the polynucleotide of the invention.

The term "vector", preferably, encompasses phage, plasmid, viral vectors as well as artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotide of the present invention, preferably, further comprises selectable markers for propagation and/or selection in a host cell. The vector may be incorporated into a host cell by various techniques well known in the art. If introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques. The terms "transformation" and "transfection", conjugation and transduction, as used in the present context, are intended to comprise a multiplicity of prior-art processes for introducing foreign nucleic acid (for example DNA) into a host cell, including calcium phosphate, rubidium chloride or calcium chloride co-precipitation, DEAE-dextran-mediated transfection, lipofection, natural competence, carbon-based clusters, chemically mediated transfer, or electroporation. Suitable methods for the transformation or transfection of host cells can be found in Sambrook et al. (Molecular Cloning: A Laboratory Manual, 2nd ed., Cold Spring Harbor Laboratory, Cold Spring Harbor Laboratory Press, Cold Spring Harbor, NY, 1989) and other laboratory manuals, such as Methods in Molecular Biology, 1995, Vol. 44, Agrobacterium protocols, Ed.: Gartland and Davey, Humana Press, Totowa, New Jersey. Alternatively, a plasmid vector may be introduced by heat shock or electroporation techniques.

Preferably, the vector referred to herein is suitable as a cloning vector, i.e. replicable in microorganisms. Such vectors ensure efficient cloning in bacteria and yeast. More preferably, the vector of the present invention is an expression vector. In such an expression vector, i.e. a vector which comprises the polynucleotide of the invention having the nucleic acid sequence operatively linked to an expression control sequence and/or a terminator (also called "expression cassette") allowing expression in prokaryotic or eukaryotic cells or isolated fractions thereof. Suitable expression vectors are known in the art such as Okayama-Berg cDNA expression vector pcDV1 (Pharmacia Biotech, Inc. US), pCDM8, pRc/CMV, pcDNA1, pcDNA3 (Invitrogene, Corp. US) or pSPORT1 (GIBCO BRL, US). Further examples of typical fusion expression vectors are pGEX (Pharmacia Biotech, Inc. US; Smith 1988, Gene 67:31-40), pMAL (New England Biolabs, US) and pRIT5 (Pharmacia Biotech, Inc. US) where glutathione S transferase (GST), maltose E-binding protein and protein A, respectively, are fused with the recombinant target protein. Examples of suitable inducible non-fusion E. coli expression vectors are, inter alia, pTrc (Amann 1988, Gene 69:301-315) pET26b(+), preferably, without pelB leader peptide, and pET 11 d (Studier 1990, Methods in Enzymology 185, 60-89). The target gene expression of the pTrc vector is based on the transcription from a hybrid trp-lac fusion promoter by host RNA polymerase. The target gene expression from the pET 11 d vector is based on the transcription of a T7gn10-lac fusion promoter, which is mediated by a co-expressed viral RNA polymerase (T7 gn1). This viral polymerase is provided by the host strains BL21 (DE3) or HMS174 (DE3) from a resident pro-phage which harbors a T7 gn1 gene under the transcriptional control of the lacUV 5 promoter. The skilled worker is familiar with other vectors which are suitable in prokaryotic organisms; these vectors are, for example, in E. coli, pLG338, pACYC184, the pBR series such as pBR322, the pUC series such as pUC18 or pUC19, the M113mp series, pKC30, pRep4, pHS1, pHS2, pPLc236, pMBL24, pLG200, pUR290, pIN-III113-B1, lambda gt11 or pBdCl, in Streptomyces pIJ101, pIJ364, pIJ702 or pIJ361, in Bacillus pUB110, pC194 or pBD214, in Corynebacterium pSA77 or pAJ667. Examples of vectors for expression in the yeast S. cerevisiae comprise pYep Sec1 (Baldari 1987, Embo J. 6:229-234), pMFa (Kurjan 1982, Cell 30:933-943), pJRY88 (Schultz 1987, Gene 54:113-123) and pYES2 (Invitrogen Corp. US). Vectors and processes for the construction of vectors which are suitable for use in other fungi, such as the filamentous fungi, comprise those which are described in detail in: van den Hondel and Punt (1991) "Gene transfer systems and vector development for filamentous fungi, in: Applied Molecular Genetics of fungi, J.F. Peberdy et al., Ed., pp. 1-28, Cambridge University Press: Cambridge, or in: More Gene Manipulations in Fungi (J.W. Bennett & L.L. Lasure, Ed., pp. 396-428: Academic Press: San Diego). Further suitable yeast vectors are, for example, pAG-1, YEp6, YEp13 or pEMBLYe23. As an alternative, the polynucleotides of the present invention may be also expressed in insect cells using baculovirus expression vectors. Baculovirus vectors which are available for the expression of proteins in cultured insect cells (for example Sf9 cells) comprise the pAc series (Smith 1983, Mol. Cell Biol. 3:2156-2165) and the pVL series (Lucklow 1989, Virology 170:31-39).

The abovementioned vectors are only a small overview of vectors to be used in accordance with the present invention. Further vectors are known to the skilled worker and are described, for example, in: Cloning Vectors (Ed., Pouwels, P.H., et al., Elsevier, Amsterdam-New York-Oxford, 1985, ISBN 0 444 904018). For further suitable expression systems for prokaryotic and eukaryotic cells see also the chapters 16 and 17 of Sambrook, loc cit.

The present invention also relates to a host cell comprising the polynucleotide or the vector of the invention.

The term "host cell" as used herein refers to a bacterium, fungus, yeast, eukaryotic cell or single- cellular organism which comprises, preferably, heterologous, the polynucleotide or the vector of the invention. The host cell may comprise the polynucleotide of the invention or the vector of the invention for the purpose of DNA propagation as well as for the purpose of expressing and manufacturing the encoded polypeptide or both. The polynucleotide or vector may be present integrated into the genome of the host cell or present in an episomal form. Suitable bacteria to be used as host cells of the invention are selected from the group consisting of: E. coli and more preferably, E. coli strain BL21-Gold (DE3), Streptomyces, Bacillus species, Corynebacteria. As referred to elsewhere herein already, such bacteria and, in particular, E. coli, Pseudomonas species or Clostridium species, can be also preferably used in the method of the present invention for degrading polyesters. Suitable fungi to be used as host cells of the invention are preferably filamentous fungi. Typically, S. cervisiae or S. pombe or Hansenula can be used as yeast in accordance with the host cell of the invention. Preferred eukaryotic cells are, e.g., insect cells in a cell culture. It will be understood that host cells as used herein encompass host cells which naturally comprise the polynucleotide of the invention and those which have been genetically modified to comprise the said polynucleotide.

Moreover, the present invention relates to a polypeptide encoded by the polynucleotide or the vector of the invention or which is obtainable by the host cell of the invention.

The term "polypeptide" as used herein encompasses purified polypeptides being essentially free of other components. However, the term also encompasses partially purified polypeptides, i.e., polypeptide preparations comprising the polypeptide of the present invention and other proteins or components in addition. A polypeptide as used herein may by a chemically modified polypeptide. Said modifications may be artificial modifications or naturally occurring modifications. The polypeptide of the present invention shall have the activities referred to above. It can be manufactured by chemical synthesis or recombinant molecular biology techniques well known for the skilled artisan. Preferably, such a method of manufacturing the polypeptide of the invention comprises (a) culturing the host cell of the present invention described elsewhere herein in more detail and (b) obtaining from the said host cell the polypeptide of the present invention. In an aspect of this method, the polypeptide can be obtained by conventional purification techniques from a lysate of the host cell including affinity chromatography, ion exchange chromatography, size exclusion chromatography and/or preparative gel electrophoresis. Details on the manufacture and testing for the desired activities are also found in the accompanying Examples below. Polypeptides according to the invention also encompass fusion proteins or polypeptide fragments being at least partially encoded by the polynucleotide of the present invention referred to above. Moreover, encompassed are chemically modified polypeptides. Such modifications may be artificial modifications or naturally occurring modifications (Review, e.g., in Mann 2003, Nat. Biotechnol. 21, 255-261).

Furthermore, the present invention relates to a composition comprising the polypeptide of the invention and a further agent which improves the degradation of polyester referred to herein and, preferably, a particularly preferred aliphatic-aromatic polyesters referred to above.

The term "composition" as used herein refers to mixture of components comprising at least the esterase polypeptide of the invention and a further agent which improves the degradation of a polyester. Such a further agent which improves the degradation of a polyester may be another enzyme for degrading the said aliphatic-aromatic polyester according to the invention. Preferably, the further agent is, therefore, a lipase and/or cutinase, preferably, obtainable from an anaerobic microorganism as specified elsewhere herein. The composition may also comprise further auxiliary components such as a solvent, preferably, comprising a buffer, enzyme stabilizers, or further compounds which facilitate the degradation of polyesters chemically or physically. Thus, the composition of the invention if applied for biogas production may, preferably, comprise also enzymes which are required for acidogenesis, acetogenesis and/or methanogenesis.

All references cited in this specification are herewith incorporated by reference with respect to their entire disclosure content and the disclosure content specifically mentioned in this specification.

### FIGURES

Figure 1 shows chromatograms that were received during HPLC analysis of the samples from aliphatic-aromatic polyester i-1 degradation experiments. (A) shows incubation with Cbotu_EstA at 50°C, 300 rpm, 0.6 µM protein for 3 days, (B) shows incubation with Chath_EstB at 37°C, 300 rpm, 6 µM proetin for 7 days, (C) shows incubation with Chath_EstA at 50°C, 550 rpm, 0.6 µM protein for 7 days.

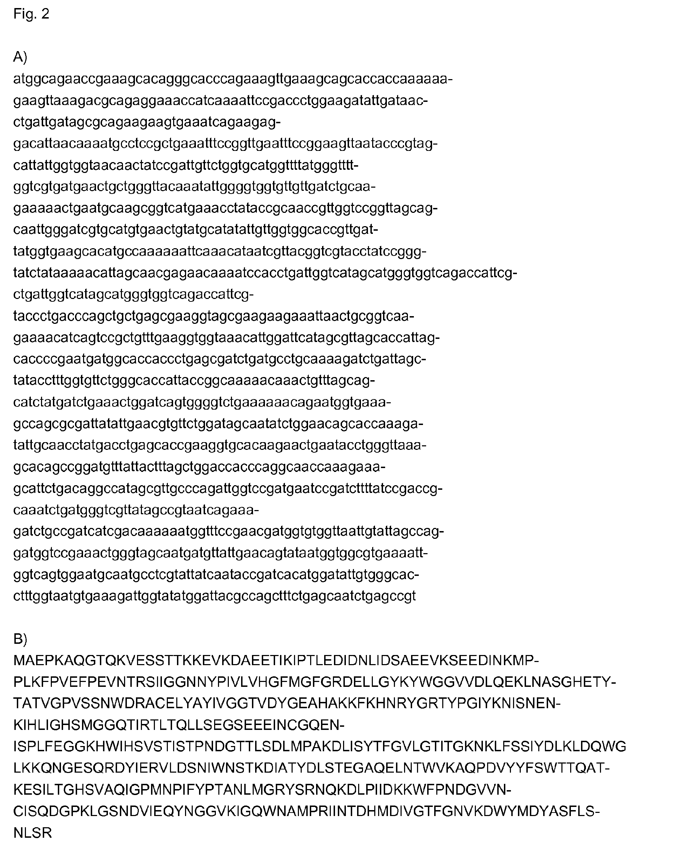

Figure 2 shows nucleic acid sequence (A) and amino acid sequence (B) of the esterase Cbotu_EstA from C. botulinum (see also SEQ ID NO: 1 and 2). The indicated nucleic acid sequence is codon-optimized for expression in E. coli.

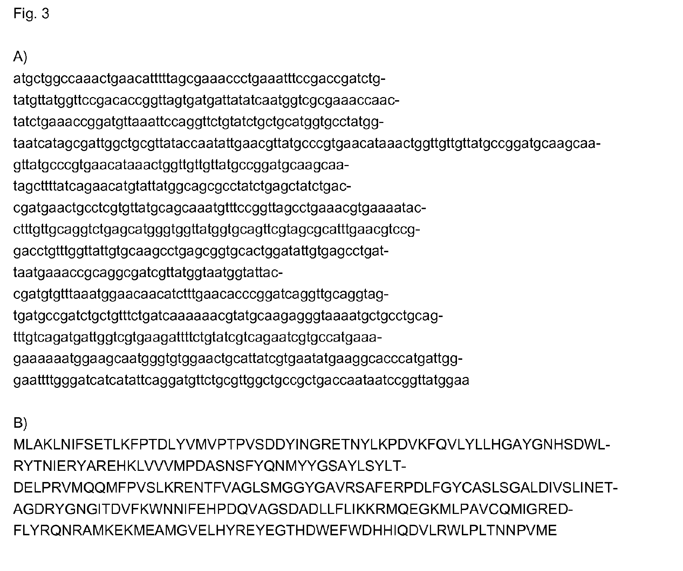

Fig. 3 shows nucleic acid sequence (A) and amino acid sequence (B) of the esterase Chath_EstAfrom C. hathewayi (see also SEQ ID NO: 3 and 4). The indicated protein sequence does not comprise a signal peptide and, thus, merely represents the mature protein. Further, the indicated nucleic acid sequence is codon-optimized for expression in E. coli.

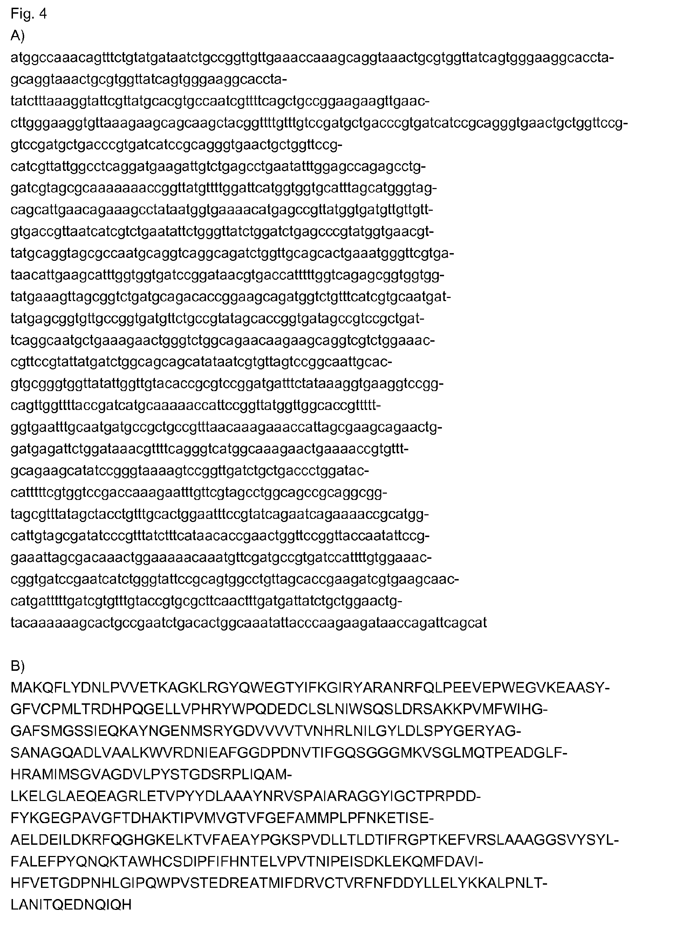

Fig. 4 shows nucleic acid sequence (A) and amino acid sequence (B) of the esterase Chath_EstB from C. hathewayi (see also SEQ ID NO: 4 and 5).

The following Examples shall merely illustrate the invention, they shall, whatsoever, not be construed in a sense limiting the scope.

### EXAMPLES

### Example 1: Enzyme expression and purification

A protein and genome database was screened for potentially interesting esterases and lipases from Clostridium by performing sequence homology searches for characteristic sequences for putative extracellular, secreted or exported lipases, esterases or hydrolases. During this in silico screening, various putative esterases and lipases were identified.

In order to proof that Clostridium species are indeed expressing esterases which degrade the aliphatic-aromatic polyester i-1 (ecoflex® F blend 1200, BASF SE, Ludwigshafen, DE), esterases from Clostridium botulinum and Clostridium hathewayi were heterologously expressed in E. coli.

To this end, the codon usage of the DNA sequences of the enzymes of interest were optimized for E. coli and on the C-terminal end a His-tag was added. After DNA synthesis, the genes were cloned into vector pET26b(+) (without pelB leader peptide) and expressed in E.coli BL21-Gold(DE3).

The expression was successful for three esterases from C. botulinum (Cbotu_EstA) and two from C. hathewayi (Chath_EstA and Chath_EstB). His-tag purification yielded in a very high protein concentration in the case of Cbotu_EstA (5.77 mg/ml) and Chath_EstB (5.4mg/ml). The expression of Chath_EstA was successful as well but protein concentration after purification was lower (0.22 mg/ml).

The standard activity assay for esterases with the soluble substrate 4-nitrophenyl butyrate was performed and revealed a very high activity of 730 U/mg for Cbotu_EstA at 25°C and pH 7. Chath_EstB exhibited an activity of 7.4 U/mg under the aforementioned conditions.

### Example 2: Degradation tests on aliphatic-aromatic polyester i-1

The expression of the three enzymes (Cbotu_EstA, Chath_EstA and Chath_EstB) worked out well and esterase activity revealed a very high activity of Cbotu_EstA and Chath_EstB for the soluble standard substrate 4-nitrophenyl butyrate. The next step was to verify that the enzymes are also active on the polyester aliphatic-aromatic polyester i-1. For that reason 10 mg of milled aliphatic-aromatic polyester i-1 were incubated in 2 ml 100 mM potassium phosphate buffer pH 7 with defined esterase concentrations. Cbotu_EstA samples were incubated with a protein concentration of 0.6 µM at 50°C and 300 rpm for 3 days, Chath_EstA samples were incubated with a protein concentration of 6 µM at 50°C and 300 rpm for 7 days. The Chath_EstB samples were incubated with a protein concentration of 0.6 µM at 37°C and 300 rpm for 7 days. Blank reactions were performed simultaneously for each enzyme under the aforementioned conditions with the difference that no esterase was added. The reactions were stopped by adding 500 µl of ice cooled methanol to 500 µl of the samples and blanks to precipitate the protein after 3 or 7 days. After incubation on ice for 15 minutes samples were centrifuged for 15 minutes at 0°C and 14000 rpm. 800 µl of the supernatant were transferred respectively to a fresh Eppendorf reaction tube and the solution was acidified by adding 2 µl of concentrated formic acid. After an incubation of 1 hour at 4°C samples were centrifuged again (15 minutes, 0°C, 14000 rpm) and 600 µl of the supernatants were transferred to HPLC vials and analyzes by means of HPLC/MS.

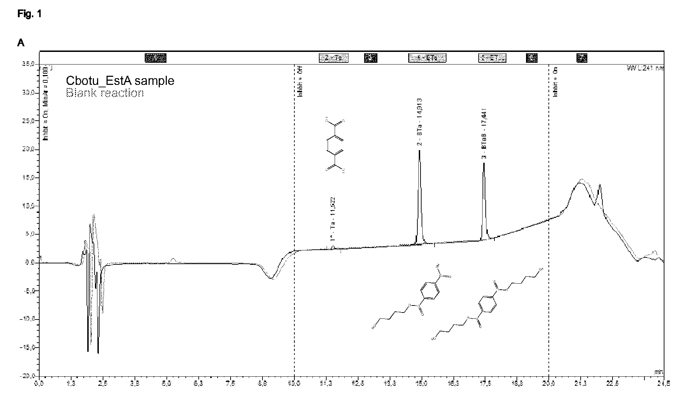

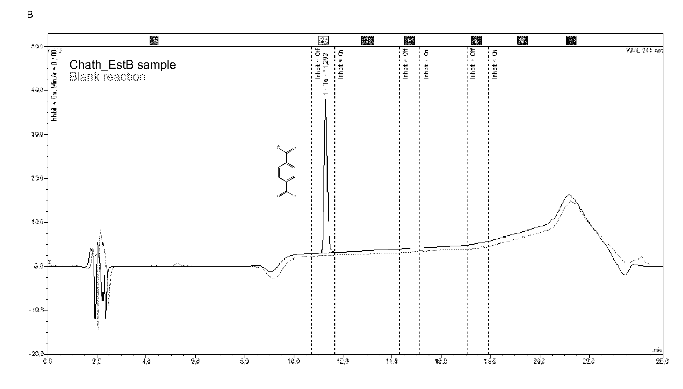

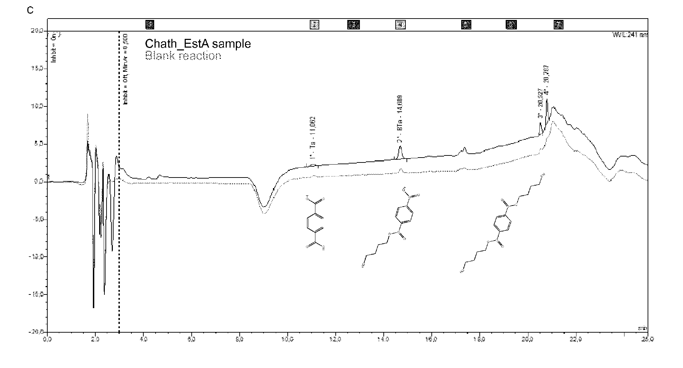

The chromatograms that were received during HPLC analysis of the samples can be found in Fig. 1.

Esterase Chath_EstA showed a very low activity on aliphatic-aromatic polyester i-1 under the given conditions. Nevertheless this does not mean that this esterase is not active on aliphaticaromatic polyester i-1 but only that expression and purification need further improvement. Furthermore the characterization of the enzyme might lead to enhanced polyester hydrolysis due to more ideal reaction conditions. (The pH and temperature optima are not yet known.). In contrast to that, esterase Chath_EstB demonstrated a notable activity on aliphatic-aromatic polyester i-1.

HPLC analysis of Cbotu_EstA and Chath_EstB samples revealed a significant hydrolysis of aliphatic-aromatic polyester i-1 if compared to the blank reaction. The hydrolysis products Ta (terephthalic acid), BTa (mono(4-hydroxybutyl) terephthalate) and BTaB (bis(4-hydroybutyl) terephthalate) were clearly detected.

In essence, the hydrolysis of aliphatic-aromatic polyester i-1 is of great interest since this polyester is produced in industrial scale and biodegradation under anaerobic condition needs to be improved. In the above described study, the potential of esterases from anaerobic organisms to hydrolyze and degrade aliphatic-aromatic polyester i-1 was clearly shown for the very first time. The proof that enzymes from anaerobic sources are able to hydrolyze aliphatic-aromatic polyester i-1 indicates that a degradation of aliphatic-aromatic polyester i-1 in biogas plants (anaerobic conditions) is also feasible.

The identification of active esterases from anaerobic sources opens up the possibility for a number of different applications. The esterases could be directly added to the biogas plants were aliphatic-aromatic polyester i-1 degradation should take place. Also the addition of certain bacterial strains to the biogas plants is an interesting option to produce the needed enzymes in situ. These two biological trigger systems might overcome the obstacle of biowaste bag residues, packaging material etc. in biogas plants. A completely different but not less interesting application of these esterases might be the use in polyester modification processes.

## Claims

1. A method for the degradation of a polyester comprising the steps of:
a) contacting the polyester with at least one esterase and/or lipase and/or cutinase, wherein the said at least one esterase and/or lipase and/or cutinase is obtainable from an microorganism; and
b) incubating the said polyester with the said at least one esterase and/or lipase and/or cutinase for a time and under conditions sufficient to allow for degradation of the polyester.

2. The method of claim 1, wherein at least the said incubating in step b) is carried out under anaerobic conditions.

3. Use of at least one esterase and/or lipase for degrading a polyester, wherein the said at least one esterase and/or lipase is obtainable from a microorganism.

4. The use of claim 3, wherein said degrading the polyester is carried out under anaerobic conditions.

5. The method of claim 1 or 2 or the use of claim 3 or 4, wherein said polyester is an aliphatic-aromatic polyester and, preferably, an aliphatic-aromatic polyester comprising
A) an acid component made of:
a1) from 30 to 100 mol% of at least one aliphatic dicarboxylic acid or ester-forming derivatives thereof, or a mixture thereof,
a2) from 0 to 70 mol% of at least one aromatic dicarboxylic acid or ester-forming derivative thereof, or a mixture thereof, and
B) from 98 to 102 mol%, based on acid component A, of a diol component B selected from at least one C2-C12-alkanediol or a mixture thereof; and
C) from 0.01 to 3% by weight, based on components A and B, of a component C selected from
c1) a compound having at least three groups capable of ester formation or of amide formation,
c2) a di- or polyisocyanate,
c3) a di- or polyepoxide, or
a mixture made of c1) to c3).

6. The method of claim 1 or 2, the use of claim 3 or 4 or the method or use of claim 5, wherein said anaerobic microorganism is a Clostridium species.

7. The method or use of any one of claims 1 to 6, wherein said esterase is encoded by a polynucleotide comprising a nucleic acid sequence selected from the group consisting of:
a) a nucleic acid sequence as shown in SEQ ID NO: 1, 3 or 5;
b) a nucleic acid sequence encoding a polypeptide having an amino acid sequence as shown in SEQ ID NO: 2, 4 or 6;
c) a nucleic acid sequence encoding a polypeptide having esterase activity, said nucleic acid sequence having a nucleic acid sequence being at least 70% identical to the nucleic acid sequence shown in SEQ ID No: 1, 3 or 5;
d) a nucleic acid sequence encoding a polypeptide having esterase activity, said polypeptide having an amino acid sequence being at least 70% identical to the amino acid sequence shown in SEQ ID No: 2, 4 or 6; and
e) a nucleic acid sequence encoding a polypeptide having esterase activity, said nucleic acid sequence being capable of hybridizing to the nucleic acid sequence of any one of a) to d).

8. The method or use of any one of claims 1 to 7, wherein said esterase and/or lipase and/or cutinase is comprised in a host cell.

9. A polynucleotide comprising a nucleic acid sequence as defined in claim 7.

10. The polynucleotide of claim 9, wherein said polynucleotide is operatively linked to an expression control sequence.

11. A vector comprising the polynucleotide of claim 9 or 10.

12. A host cell comprising the polynucleotide of claim 9 or 10 or the vector of claim 11.

13. A polypeptide encoded by the polynucleotide of claim 9 or 10, the vector of claim 11 or obtainable by the host cell of claim 12.

14. A composition comprising the polypeptide of claim 13 and a further agent which improves the degradation of degrading a polyester.

15. The composition of claim 14, wherein said further agent is a lipase and/or cutinase, preferably, obtainable from an anaerobic microorganism.


SEQUENCE LISTING

<110> BASF SE

<120> Degradation of polyesters under anaerobic conditions

<130> B75278EP

<160> 6

<170> BiSSAP 1.0

<210> 1
<211> 1356
<212> DNA
<213> Clostridium botulinum

<220>
<221> source
<222> 1..1356
<223> /mol_type="DNA"
      /note="Cbotu_EstA "
      /organism="Clostridium botulinum"

<400> 1
atggcagaac cgaaagcaca gggcacccag aaagttgaaa gcagcaccac caaaaaagaa      60

gttaaagacg cagaggaaac catcaaaatt ccgaccctgg aagatattga taacctgatt     120

gatagcgcag aagaagtgaa atcagaagag gacattaaca aaatgcctcc gctgaaattt     180

ccggttgaat ttccggaagt taatacccgt agcattattg gtggtaacaa ctatccgatt     240

gttctggtgc atggttttat gggttttggt cgtgatgaac tgctgggtta caaatattgg     300

ggtggtgttg ttgatctgca agaaaaactg aatgcaagcg gtcatgaaac ctataccgca     360

accgttggtc cggttagcag caattgggat cgtgcatgtg aactgtatgc atatattgtt     420

ggtggcaccg ttgattatgg tgaagcacat gccaaaaaat tcaaacataa tcgttacggt     480

cgtacctatc cgggtatcta taaaaacatt agcaacgaga acaaaatcca cctgattggt     540

catagcatgg gtggtcagac cattcgtacc ctgacccagc tgctgagcga aggtagcgaa     600

gaagaaatta actgcggtca agaaaacatc agtccgctgt ttgaaggtgg taaacattgg     660

attcatagcg ttagcaccat tagcaccccg aatgatggca ccaccctgag cgatctgatg     720

cctgcaaaag atctgattag ctataccttt ggtgttctgg gcaccattac cggcaaaaac     780

aaactgttta gcagcatcta tgatctgaaa ctggatcagt ggggtctgaa aaaacagaat     840

ggtgaaagcc agcgcgatta tattgaacgt gttctggata gcaatatctg gaacagcacc     900

aaagatattg caacctatga cctgagcacc gaaggtgcac aagaactgaa tacctgggtt     960

aaagcacagc cggatgttta ttactttagc tggaccaccc aggcaaccaa agaaagcatt    1020

ctgacaggcc atagcgttgc ccagattggt ccgatgaatc cgatctttta tccgaccgca    1080

aatctgatgg gtcgttatag ccgtaatcag aaagatctgc cgatcatcga caaaaaatgg    1140

tttccgaacg atggtgtggt taattgtatt agccaggatg gtccgaaact gggtagcaat     1200

gatgttattg aacagtataa tggtggcgtg aaaattggtc agtggaatgc aatgcctcgt     1260

attatcaata ccgatcacat ggatattgtg ggcacctttg gtaatgtgaa agattggtat     1320

atggattacg ccagctttct gagcaatctg agccgt                               1356

<210> 2
<211> 452
<212> PRT
<213> Clostridium botulinum

<220>
<221> SOURCE
<222> 1..452
<223> /mol_type="protein"
      /note="Cbotu_EstA prt"
      /organism="Clostridium botulinum"

<400> 2
Met Ala Glu Pro Lys Ala Gln Gly Thr Gln Lys Val Glu Ser Ser Thr
1               5                   10                  15
Thr Lys Lys Glu Val Lys Asp Ala Glu Glu Thr Ile Lys Ile Pro Thr
            20                  25                  30
Leu Glu Asp Ile Asp Asn Leu Ile Asp Ser Ala Glu Glu Val Lys Ser
        35                  40                  45
Glu Glu Asp Ile Asn Lys Met Pro Pro Leu Lys Phe Pro Val Glu Phe
    50                  55                  60
Pro Glu Val Asn Thr Arg Ser Ile Ile Gly Gly Asn Asn Tyr Pro Ile
65                  70                  75                  80
Val Leu Val His Gly Phe Met Gly Phe Gly Arg Asp Glu Leu Leu Gly
                85                  90                  95
Tyr Lys Tyr Trp Gly Gly Val Val Asp Leu Gln Glu Lys Leu Asn Ala
            100                 105                 110
Ser Gly His Glu Thr Tyr Thr Ala Thr Val Gly Pro Val Ser Ser Asn
        115                 120                 125
Trp Asp Arg Ala Cys Glu Leu Tyr Ala Tyr Ile Val Gly Gly Thr Val
    130                 135                 140
Asp Tyr Gly Glu Ala His Ala Lys Lys Phe Lys His Asn Arg Tyr Gly
145                 150                 155                 160
Arg Thr Tyr Pro Gly Ile Tyr Lys Asn Ile Ser Asn Glu Asn Lys Ile
                165                 170                 175
His Leu Ile Gly His Ser Met Gly Gly Gln Thr Ile Arg Thr Leu Thr
            180                 185                 190
Gln Leu Leu Ser Glu Gly Ser Glu Glu Glu Ile Asn Cys Gly Gln Glu
        195                 200                 205
Asn Ile Ser Pro Leu Phe Glu Gly Gly Lys His Trp Ile His Ser Val
    210                 215                 220
Ser Thr Ile Ser Thr Pro Asn Asp Gly Thr Thr Leu Ser Asp Leu Met
225                 230                 235                 240
Pro Ala Lys Asp Leu Ile Ser Tyr Thr Phe Gly Val Leu Gly Thr Ile
                245                 250                 255
Thr Gly Lys Asn Lys Leu Phe Ser Ser Ile Tyr Asp Leu Lys Leu Asp
            260                 265                 270
Gln Trp Gly Leu Lys Lys Gln Asn Gly Glu Ser Gln Arg Asp Tyr Ile
        275                 280                 285
Glu Arg Val Leu Asp Ser Asn Ile Trp Asn Ser Thr Lys Asp Ile Ala
    290                 295                 300
Thr Tyr Asp Leu Ser Thr Glu Gly Ala Gln Glu Leu Asn Thr Trp Val
305                 310                 315                 320
Lys Ala Gln Pro Asp Val Tyr Tyr Phe Ser Trp Thr Thr Gln Ala Thr

325                 330                 335
Lys Glu Ser Ile Leu Thr Gly His Ser Val Ala Gln Ile Gly Pro Met
            340                 345                 350
Asn Pro Ile Phe Tyr Pro Thr Ala Asn Leu Met Gly Arg Tyr Ser Arg
        355                 360                 365
Asn Gln Lys Asp Leu Pro Ile Ile Asp Lys Lys Trp Phe Pro Asn Asp
    370                 375                 380
Gly Val Val Asn Cys Ile Ser Gln Asp Gly Pro Lys Leu Gly Ser Asn
385                 390                 395                 400
Asp Val Ile Glu Gln Tyr Asn Gly Gly Val Lys Ile Gly Gln Trp Asn
                405                 410                 415
Ala Met Pro Arg Ile Ile Asn Thr Asp His Met Asp Ile Val Gly Thr
            420                 425                 430
Phe Gly Asn Val Lys Asp Trp Tyr Met Asp Tyr Ala Ser Phe Leu Ser
        435                 440                 445
Asn Leu Ser Arg
    450

<210> 3
<211> 825
<212> DNA
<213> Clostridium hathewayi

<220>
<221> source
<222> 1..825
<223> /mol_type="DNA"
      /note="Chath_EstA"
      /organism="Clostridium hathewayi"

<400> 3
atgctggcca aactgaacat ttttagcgaa accctgaaat ttccgaccga tctgtatgtt      60

atggttccga caccggttag tgatgattat atcaatggtc gcgaaaccaa ctatctgaaa     120

ccggatgtta aattccaggt tctgtatctg ctgcatggtg cctatggtaa tcatagcgat     180

tggctgcgtt ataccaatat tgaacgttat gcccgtgaac ataaactggt tgttgttatg     240

ccggatgcaa gcaatagctt ttatcagaac atgtattatg gcagcgccta tctgagctat     300

ctgaccgatg aactgcctcg tgttatgcag caaatgtttc cggttagcct gaaacgtgaa     360

aatacctttg ttgcaggtct gagcatgggt ggttatggtg cagttcgtag cgcatttgaa     420

cgtccggacc tgtttggtta ttgtgcaagc ctgagcggtg cactggatat tgtgagcctg     480

attaatgaaa ccgcaggcga tcgttatggt aatggtatta ccgatgtgtt taaatggaac     540

aacatctttg aacacccgga tcaggttgca ggtagtgatg ccgatctgct gtttctgatc     600

aaaaaacgta tgcaagaggg taaaatgctg cctgcagttt gtcagatgat tggtcgtgaa     660

gattttctgt atcgtcagaa tcgtgccatg aaagaaaaaa tggaagcaat gggtgtggaa     720

ctgcattatc gtgaatatga aggcacccat gattgggaat tttgggatca tcatattcag     780

gatgttctgc gttggctgcc gctgaccaat aatccggtta tggaa                     825


<210> 4
<211> 275
<212> PRT

<213> Clostridium hathewayi

<220>
<221> SOURCE
<222> 1..275
<223> /mol_type="protein"
      /note="Cath_EstA prt"
      /organism="Clostridium hathewayi"

<400> 4
Met Leu Ala Lys Leu Asn Ile Phe Ser Glu Thr Leu Lys Phe Pro Thr
1               5                   10                  15
Asp Leu Tyr Val Met Val Pro Thr Pro Val Ser Asp Asp Tyr Ile Asn
            20                  25                  30
Gly Arg Glu Thr Asn Tyr Leu Lys Pro Asp Val Lys Phe Gln Val Leu
        35                  40                  45
Tyr Leu Leu His Gly Ala Tyr Gly Asn His Ser Asp Trp Leu Arg Tyr
    50                  55                  60
Thr Asn Ile Glu Arg Tyr Ala Arg Glu His Lys Leu Val Val Val Met
65                  70                  75                  80
Pro Asp Ala Ser Asn Ser Phe Tyr Gln Asn Met Tyr Tyr Gly Ser Ala
                85                  90                  95
Tyr Leu Ser Tyr Leu Thr Asp Glu Leu Pro Arg Val Met Gln Gln Met
            100                 105                 110
Phe Pro Val Ser Leu Lys Arg Glu Asn Thr Phe Val Ala Gly Leu Ser
        115                 120                 125
Met Gly Gly Tyr Gly Ala Val Arg Ser Ala Phe Glu Arg Pro Asp Leu
    130                 135                 140
Phe Gly Tyr Cys Ala Ser Leu Ser Gly Ala Leu Asp Ile Val Ser Leu
145                 150                 155                 160
Ile Asn Glu Thr Ala Gly Asp Arg Tyr Gly Asn Gly Ile Thr Asp Val
                165                 170                 175
Phe Lys Trp Asn Asn Ile Phe Glu His Pro Asp Gln Val Ala Gly Ser
            180                 185                 190
Asp Ala Asp Leu Leu Phe Leu Ile Lys Lys Arg Met Gln Glu Gly Lys
        195                 200                 205
Met Leu Pro Ala Val Cys Gln Met Ile Gly Arg Glu Asp Phe Leu Tyr
    210                 215                 220
Arg Gln Asn Arg Ala Met Lys Glu Lys Met Glu Ala Met Gly Val Glu
225                 230                 235                 240
Leu His Tyr Arg Glu Tyr Glu Gly Thr His Asp Trp Glu Phe Trp Asp
                245                 250                 255
His His Ile Gln Asp Val Leu Arg Trp Leu Pro Leu Thr Asn Asn Pro
            260                 265                 270
Val Met Glu
        275

<210> 5
<211> 1557
<212> DNA
<213> Clostridium hathewayi

<220>
<221> source
<222> 1..1557
<223> /mol_type="DNA"
      /note="Cath_EstB"
      /organism="Clostridium hathewayi"

<400> 5
atggccaaac agtttctgta tgataatctg ccggttgttg aaaccaaagc aggtaaactg      60

cgtggttatc agtgggaagg cacctatatc tttaaaggta ttcgttatgc acgtgccaat      120

cgttttcagc tgccggaaga agttgaacct tgggaaggtg ttaaagaagc agcaagctac 180

ggttttgttt gtccgatgct gacccgtgat catccgcagg gtgaactgct ggttccgcat 240

cgttattggc ctcaggatga agattgtctg agcctgaata tttggagcca gagcctggat 300

cgtagcgcaa aaaaaccggt tatgttttgg attcatggtg gtgcatttag catgggtagc 360

agcattgaac agaaagccta taatggtgaa aacatgagcc gttatggtga tgttgttgtt 420

gtgaccgtta atcatcgtct gaatattctg ggttatctgg atctgagccc gtatggtgaa 480

cgttatgcag gtagcgccaa tgcaggtcag gcagatctgg ttgcagcact gaaatgggtt 540

cgtgataaca ttgaagcatt tggtggtgat ccggataacg tgaccatttt tggtcagagc 600

ggtggtggta tgaaagttag cggtctgatg cagacaccgg aagcagatgg tctgtttcat 660

cgtgcaatga ttatgagcgg tgttgccggt gatgttctgc cgtatagcac cggtgatagc 720

cgtccgctga ttcaggcaat gctgaaagaa ctgggtctgg cagaacaaga agcaggtcgt 780

ctggaaaccg ttccgtatta tgatctggca gcagcatata atcgtgttag tccggcaatt 840

gcacgtgcgg gtggttatat tggttgtaca ccgcgtccgg atgatttcta taaaggtgaa 900

ggtccggcag ttggttttac cgatcatgca aaaaccattc cggttatggt tggcaccgtt 960

tttggtgaat ttgcaatgat gccgctgccg tttaacaaag aaaccattag cgaagcagaa 1020

ctggatgaga ttctggataa acgttttcag ggtcatggca aagaactgaa aaccgtgttt 1080

gcagaagcat atccgggtaa aagtccggtt gatctgctga ccctggatac catttttcgt 1140

ggtccgacca aagaatttgt tcgtagcctg gcagccgcag gcggtagcgt ttatagctac 1200

ctgtttgcac tggaatttcc gtatcagaat cagaaaaccg catggcattg tagcgatatc 1260

ccgtttatct ttcataacac cgaactggtt ccggttacca atattccgga aattagcgac 1320

aaactggaaa aacaaatgtt cgatgccgtg atccattttg tggaaaccgg tgatccgaat 1380

catctgggta ttccgcagtg gcctgttagc accgaagatc gtgaagcaac catgattttt 1440

gatcgtgttt gtaccgtgcg cttcaacttt gatgattatc tgctggaact gtacaaaaaa 1500

gcactgccga atctgacact ggcaaatatt acccaagaag ataaccagat tcagcat 1557

<210> 6
<211> 519
<212> PRT
<213> Clostridium hathewayi

<220>
<221> SOURCE
<222> 1..519
<223> /mol_type="protein"
      /note="Cath_EstB prt"
      /organism="Clostridium hathewayi"

<400> 6

Met Ala Lys Gln Phe Leu Tyr Asp Asn Leu Pro Val Val Glu Thr Lys
1               5                   10                  15
Ala Gly Lys Leu Arg Gly Tyr Gln Trp Glu Gly Thr Tyr Ile Phe Lys
            20                  25                  30
Gly Ile Arg Tyr Ala Arg Ala Asn Arg Phe Gln Leu Pro Glu Glu Val
        35                  40                  45
Glu Pro Trp Glu Gly Val Lys Glu Ala Ala Ser Tyr Gly Phe Val Cys
    50                  55                  60
Pro Met Leu Thr Arg Asp His Pro Gln Gly Glu Leu Leu Val Pro His
65                  70                  75                  80
Arg Tyr Trp Pro Gln Asp Glu Asp Cys Leu Ser Leu Asn Ile Trp Ser
                85                  90                  95
Gln Ser Leu Asp Arg Ser Ala Lys Lys Pro Val Met Phe Trp Ile His
            100                 105                 110
Gly Gly Ala Phe Ser Met Gly Ser Ser Ile Glu Gln Lys Ala Tyr Asn
        115                 120                 125
Gly Glu Asn Met Ser Arg Tyr Gly Asp Val Val Val Val Thr Val Asn
    130                 135                 140
His Arg Leu Asn Ile Leu Gly Tyr Leu Asp Leu Ser Pro Tyr Gly Glu
145                 150                 155                 160
Arg Tyr Ala Gly Ser Ala Asn Ala Gly Gln Ala Asp Leu Val Ala Ala
                165                 170                 175
Leu Lys Trp Val Arg Asp Asn Ile Glu Ala Phe Gly Gly Asp Pro Asp
            180                 185                 190
Asn Val Thr Ile Phe Gly Gln Ser Gly Gly Gly Met Lys Val Ser Gly
        195                 200                 205
Leu Met Gln Thr Pro Glu Ala Asp Gly Leu Phe His Arg Ala Met Ile
    210                 215                 220
Met Ser Gly Val Ala Gly Asp Val Leu Pro Tyr Ser Thr Gly Asp Ser
225                 230                 235                 240
Arg Pro Leu Ile Gln Ala Met Leu Lys Glu Leu Gly Leu Ala Glu Gln
                245                 250                 255
Glu Ala Gly Arg Leu Glu Thr Val Pro Tyr Tyr Asp Leu Ala Ala Ala
            260                 265                 270
Tyr Asn Arg Val Ser Pro Ala Ile Ala Arg Ala Gly Gly Tyr Ile Gly
        275                 280                 285
Cys Thr Pro Arg Pro Asp Asp Phe Tyr Lys Gly Glu Gly Pro Ala Val
    290                 295                 300
Gly Phe Thr Asp His Ala Lys Thr Ile Pro Val Met Val Gly Thr Val
305                 310                 315                 320
Phe Gly Glu Phe Ala Met Met Pro Leu Pro Phe Asn Lys Glu Thr Ile
                325                 330                 335
Ser Glu Ala Glu Leu Asp Glu Ile Leu Asp Lys Arg Phe Gln Gly His
            340                 345                 350
Gly Lys Glu Leu Lys Thr Val Phe Ala Glu Ala Tyr Pro Gly Lys Ser
        355                 360                 365
Pro Val Asp Leu Leu Thr Leu Asp Thr Ile Phe Arg Gly Pro Thr Lys
    370                 375                 380
Glu Phe Val Arg Ser Leu Ala Ala Ala Gly Gly Ser Val Tyr Ser Tyr
385                 390                 395                 400
Leu Phe Ala Leu Glu Phe Pro Tyr Gln Asn Gln Lys Thr Ala Trp His
                405                 410                 415
Cys Ser Asp Ile Pro Phe Ile Phe His Asn Thr Glu Leu Val Pro Val
            420                 425                 430
Thr Asn Ile Pro Glu Ile Ser Asp Lys Leu Glu Lys Gln Met Phe Asp
        435                 440                 445
Ala Val Ile His Phe Val Glu Thr Gly Asp Pro Asn His Leu Gly Ile
    450                 455                 460
Pro Gln Trp Pro Val Ser Thr Glu Asp Arg Glu Ala Thr Met Ile Phe
465                 470                 475                 480
Asp Arg Val Cys Thr Val Arg Phe Asn Phe Asp Asp Tyr Leu Leu Glu
                485                 490                 495
Leu Tyr Lys Lys Ala Leu Pro Asn Leu Thr Leu Ala Asn Ile Thr Gln

            500                 505                 510
Glu Asp Asn Gln Ile Gln His
        515